(19) Europäisches Patentamt
European Patent Office
Office européen des brevets

(11) Numéro de publication: **0 055 951**
**B1**

(12) **FASCICULE DE BREVET EUROPEEN**

(45) Date de publication du fascicule du brevet: **30.05.84**

(51) Int. Cl.³: **C 07 C 50/16**, C 07 C 50/24, C 07 C 46/00

(21) Numéro de dépôt: **81401932.9**

(22) Date de dépôt: **04.12.81**

(54) **Procédé pour la préparation de l'anthraquinone et de ses dérivés substitués.**

(30) Priorité: **16.12.80 FR 8026637**

(43) Date de publication de la demande: **14.07.82 Bulletin 82/28**

(45) Mention de la délivrance du brevet: **30.05.84 Bulletin 84/22**

(84) Etats contractants désignés: **BE CH DE FR GB IT LI**

(56) Documents cités:
**DE - A - 2 031 430**
**DE - C - 634 987**
**FR - A - 2 262 007**
**FR - A - 2 307 786**
**US - A - 2 174 118**
**US - A - 2 871 244**

(73) Titulaire: **Société ATOCHEM, 12-16, Allée des Vosges, F-92400 Courbevoie (FR)**

(72) Inventeur: **Devic, Michel, 134, rue Edmond Locard, F-69005 Lyon (FR)**

(74) Mandataire: **Rochet, Michel et al, ATOCHEM Service PROPRIETE INDUSTRIELLE Tour Manhattan Cédex 21, F-92091 Paris la Défense (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 055 951 B1

ACTORUM AG

## Description

L'invention a pour objet un nouveau procédé pour la préparation de l'anthraquinone et de ses dérivés substitués de formule générale:

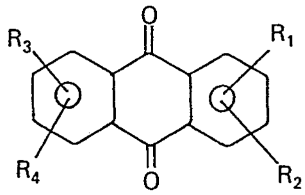

(I)

dans laquelle $R_1$, $R_2$, $R_3$, $R_4$, représentent un atome d'hydrogéne ou d'halogène ou un groupe alkyle linéaire ou ramifié possédant 1 à 5 atomes de carbone. Ces composés sont utiles pour l'industrie des matières colorantes, des pâtes à papier et pour la fabrication de l'eau oxygénée.

On sait que l'anthraquinone est produite industriellement par oxydation de l'anthracène (BIOS 1148). Ce procédé cependant est tributaire des problèmes d'approvisionnement en anthracène issu du goudron de houille.

Il a été proposé de préparer l'anthraquinone à partir de la naphtoquinone-1,4, et du butadiène (BP 895 620) mais les procédés d'obtention de la naphtoquinone-1,4 sont compliqués et coûteux (DOS 2 532 365).

Une autre méthode industrielle très employée permet de préparer l'anthraquinone et les anthraquinones substituées à partir d'anhydride phtalique et de benzène en présence de chlorure d'aluminium (US 1 656 575), mais cette méthode est pénalisée par le coût très important du chlorure d'aluminium qui est consommé à raison de 2 moles de chlorure d'aluminium par mole d'anhydride phtalique.

Pour pallier cet inconvénient il a été proposé de faire réagir le mélange gazeux à haute température sur un catalyseur solide à base de silico-aluminate (Japan Kokai Sho 49.30350 et Sho 49.95952) ou bien à base d'oxyde de titane (Japan Kokai Sho 54.70252) mais ces procédés imposent d'opérer en phase gazeuse à haute température et de nécessiter une installation complexe d'un investissement coûteux.

Dans ces conditions la demanderesse s'est assignée pour but de trouver un catalyseur permettant d'effectuer la condensation de l'anhydride phtalique sur le benzène à basse température et en phase liquide comme le chlorure d'aluminium, mais présentant l'avantage de pouvoir être récupéré après la réaction.

Il a déjà été montré (USRR 189 445) que l'acide fluorhydrique catalyse la condensation de l'anhydride phtalique sur les phénols; mais cette réaction n'a lieu qu'avec les dérivés du benzène fortement activés par la fonction phénol et aucune condensation ne se produit avec le benzène, le chlorobenzène et les alkyl-benzènes. Le trifluorure de bore ne présente pas non plus une activité catalytique suffisante pour condenser le benzène avec l'anhydride phtalique.

C'est alors qu'il a été découvert, conformément à la présente invention effectuée dans les services de la demanderesse, que le mélange équimolaire d'HF et de $BF_3$ catalysait la condensation de l'anhydride phtalique sur le benzène et cela – fait surprenant – sans provoquer la cyclisation de l'acide benzoyl-benzoïque formé, donc sans libérer une molécule d'eau qui aurait hydrolysé ou hydraté $BF_3$ rendant ainsi la récupération du catalyseur difficile.

Le fait que le mélange équimolaire HF et $BF_3$ ne provoque pas de cyclisation est tout à fait inattendu car la cyclisation de l'acide benzoyl-benzoïque a lieu dans HF anhydre (US patent no 2 174 118).

La présente invention concerne donc un procédé pour la préparation des composés de formule générale (I) qui consiste à faire réagir l'anhydride phtalique, éventuellement substitué, de formule:

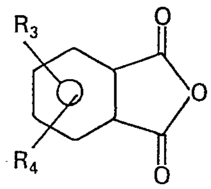

(II)

avec un composé benzénique de formule générale:

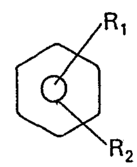

(III)

dans lesquelles $R_1$, $R_2$, $R_3$, $R_4$, ont les mêmes significtions que ci-dessus, en présence d'un catalyseur, caractérisé en ce que le catalyseur est constitué par un mélange d'acide fluorhydrique et de trifluorure de bore tel que la quantité de trifluorure de bore est supérieure à 3 moles par mole d'anhydride phtalique, la quantité d'acide fluorhydrique supérieure à 2 moles par mole d'anhydride phtalique, le rapport molaire acide fluorhydrique/trifluorure de bore compris entre 0,2 et 1,5, et en ce que le rapport molaire dérivé aromatique de formule (III)/anhydride phtalique est égal à 0,9 à 1,2, l'acide orthobenzoylbenzoïque ainsi obtenu de formule générale:

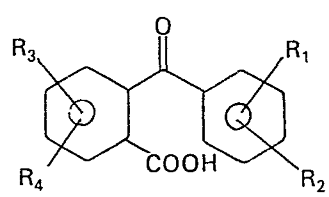

(IV)

dans laquelle $R_1$, $R_2$, $R_3$, $R_4$ ont les mêmes significations que ci-dessus, étant transformé en un composé de formule (I) suivant des procédés connus en soi.

Le rapport molaire HF sur $BF_3$ doit être voisin de 1 lorsque le catalyseur est employé en quantité minimum (somme du nombre de mole d'HF et de $BF_3$ inférieure à 15). Le rapport préféré est alors compris entre 0,6 et 1,5. Pour des quantités de $BF_3$ supérieures à 10 moles par mole d'anhydride phtalique, le rapport préféré est compris entre 0,2 et 1.

Pour diminuer la formation de sous-produit, il est préférable d'utiliser 1 à 1,1 mole de dérivé aromatique de formule III par mole d'anhydride phtalique. Un excès benzénique favorise la formation de dérivés phénylptalide de formules générales:

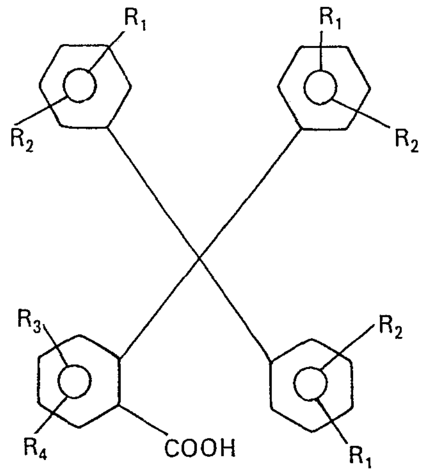

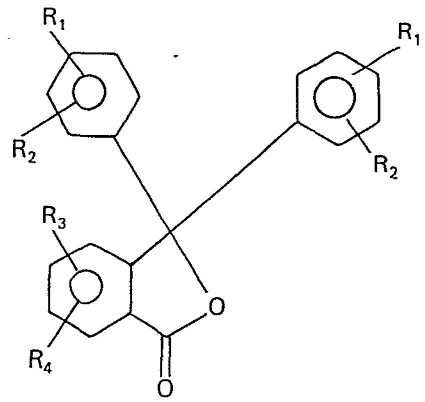

Le catalyseur tenant également lieu de solvant de la réaction, il doit être utilisé en quantité suffisante pour fluidifier la masse de réaction, soit 5 à 20 moles de $BF_3$ et d'HF par mole d'anhydride phtalique. Cette quantité peut être réduite par l'emploi d'un tiers solvant inerte dans les conditions de la réaction: comme solvants inertes on peut utiliser le chlorure de méthylène et $CO_2$ liquide.

La réaction peut être effectuée à des températures allant de −60°C à +30°C, le domaine des températures préférées étant de −40°C à 0°C. Une température supérieure à 30°C augmente la quantité de sous-produit du type phényl-phtalide.

La durée de la réaction est comprise entre 5 et 60 minutes selon la température. La réaction a lieu sous une pression de 5 à 60 bars selon la température.

En fin de réaction la plus grande partie du catalyseur est récupérée par distillation sous pression réduite.

L'acide o.benzoyl benzoïque pur est obtenu par extraction du mélagne brut par de l'eau bouillante puis recristallisation par refroidissement. Les impuretés de la réaction forment une phase non miscible à l'eau et peuvent être éliminées par décantation ou par filtration sur un support absorbant.

L'acide o.benzoyl benzoïque pur peut aussi être obtenu en traitant le mélange réactionnel, après évaporation sous vide du catalyseur, par une solution aqueuse de soude puis reprécipitation par un acide minéral.

L'acide o.benzoyl benzoïque de formule générale (IV) peut être transformé en anthraquinone de formule générale (I) par chauffage dans l'acide sulfurique concentré ou bien par tout autre moyen de cyclisation connu du chimiste.

C'est ainsi que dans la fabrication des intermédiaires anthraquinoniques pour colorant, la cyclisation peut avoir lieu au cours des réactions de sulfonation et de nitration qui ont lieu dans un milieu cyclisant comme par exemple $H_2SO_4$ ou HF.

Les exemples suivants illustrant la présente invention sans la limiter.

Exemple I

Dans un autoclave en acier inox on charge à température ordinaire 7,4 g d'anhydride phtalique et 3,9 g de benzène. L'autoclave est refroidi par de l'azote liquide et on introduit 10 g d'HF et 37,4 g de $BF_3$. On réchauffe l'autoclave à 20°C; la pression s'élève à 47 bars. On maintient 15 minutes à 20°C et dégaze sous pression réduite pour récupérer le catalyseur.

On obtient après lavage à l'eau froide 10 g d'acide o.benzoyl benzoïque brut titrant 74,4% et contenant 0,5% d'anthraquinone. Soit un rendement chimique de 66%. Après purification par extraction avec 600 cm³ d'eau bouillante puis cristallisation par refroidissement à 20°C, on obtient de l'acide o.benzoyl benzoïque titrant 98,4%.

5 g de l'acide benzoyl benzoïque purifié sont chauffés à 100°C pendant 2 heures dans 50 g d'acide sulfurique fumant (20% $SO_3$). Le mélange est coulé sur 350 cm³ d'eau et de glace et le précipité obtenu est est filtré lavé et séché.

On obtient 4,2 g d'anthraquinone.

Exemple 2 (contradictoire)

On procède comme à l'exemple 1, mais avec 7,4 g d'anhydride phtalique, 3,9 g de benzène, 85 g de $BF_3$ et pas d'HF. On maintient à 60°C pendant 4 heures sous 22 bars. Après évaporation sous vide du catalyseur, le résidu est lavé à l'eau froide puis séché. On obtient 5,6 g de produit constitué essentiellement d'acide phtalique.

Exemple 3 (contradictoire)

On procède selon l'exemple 1, mais avec 7,4 g d'anhydride phtalique, 3,9 g de benzène, 10 g d'HF et pas de $BF_3$. On maintient à 100°C pendant 4 heures sous 5 bars. Après évaporation sous vide du catalyseur, le résidu est lavé à l'eau froide puis séché. On obtient 7,01 g de produit constitué essentiellement d'acide phtalique.

Exemple 4 (contradictoire)

On procède exactement comme à l'exemple 1, mais en mettant une quantité double de benzène. On obtient 7,5 g d'un produit contenant seulement 0,3% d'acide benzoyl benzoïque et 1% d'anthraquinone.

Exemple 5

Dans un autoclave en acier inox on charge à température ordinaire 7,4 g d'anhydride phtalique et 5,3 g d'éthyl-benzène. L'autoclave est refroidi par de l'azote liquide et on introduit 10 g d'HF et 37,4 g de $BF_3$. On réchauffe l'autoclave à environ 0°C pour porter la pression à 20 bars. On maintient 15 mn à cette pression puis dégaze sous pression réduite pour récupérer le catalyseur. Le résidu est lavé à l'eau froide et séché.

On obtient 9,4 g d'acide éthyl-benzoyl-benzoïque brut titrant 81%, soit un rendement chimique de 60%.

Après purification par extraction à l'eau bouillante, puis cristallisation par refroidissement on obtient de l'acide éthyl-benzoyl-benzoïque. 5 g de ce produit sont chauffés dans de l'oléum 20%, selon l'exemple 1. On obtient 3,75 g de 2-éthyl-anthraquinone.

Exemple 6

Dans un autoclave en acier inox on charge à température ordinaire 22,2 g d'anhydride phtalique (0,15 mole). L'autoclave est refroidi par de l'azote liquide. On introduit 30 g d'HF (1,5 mole) et 102 g de $BF_3$ (1,5 mole), porte la température à −40°C et introduit en 15 mn 12,9 g de benzène (0,165 mole). On maintient 1 heure à −40°C sous 16 bars, décomprime à −40°C puis dégaze sous vide à +20°C pour évaporer HF et $BF_3$. Le solide obtenu est de l'acide o.benzoyl benzoïque (O.B.B.) contenant un peu d'HF et de $BF_3$.

L'acide O.B.B. brut est dissout à froid dans de la soude à 10%. La solution est filtrée. On précipite l'acide O.B.B. par addition d'HCl concentré. On obtient 30,6 g d'acide O.B.B. à 94% de pureté (85% de rendement chimique).

5 g de cet acide O.B.B. sont chauffés à 100°C pendant 2 heures dans 50 g d'acide sulfurique fumant (20% $SO_3$). Le mélange est coulé sur 350 cm³ d'eau. Le précipité obtenu est filtré, lavé et séché. On obtient 4,10 g d'anthraquinone à 99,7% de pureté.

Exemple 7

On opère comme à l'exemple 6 mais avec plus de $BF_3$ et moins d'HF, soit 15 g d'HF (0,75 mole) et 153 g de $BF_3$ (2,25 moles). On obtient 30,0 g d'acide de O.B.B. à 93,7% (83% de rendement chimique). 5 g de cet acide O.B.B. donnent par chauffage dans l'acide sulfurique 4,10 g d'anthraquinone.

Exemple 8

On opère comme à l'exemple 7 mais en remplaçant le benzène par 13,55 g de monochlorobenzène (0,165 mole). On obtient 12,6 g d'acide chloro-benzoyl-benzoïque pur (32% de rendement chimique). Par chauffage à 150°C dans de l'acide sulfurique à 100%, l'acide chloro-benzoylbenzoïque obtenu donne de la chloro-2 anthraquinone.

Exemple 9

On opère comme à l'exemple 6 mais avec une quantité plus grande de catalyseur soit 45 g d'HF (2,25 moles) et 153 g de $BF_3$ (2,25 moles). On obtient 40,1 g d'acide O.B.B. brut à 76,2% de pureté (rendement chimique de 90%). 5 g de cet acide O.B.B. donnent par chauffage dans l'acide sulfurique 3,35 g d'anthraquinone.

Exemple 10

On opère comme à l'exemple 7 mais en remplaçant le benzène par 17,5 g d'éthylbenzène (0,165 mole). On obtient 33,7 g d'acide éthylbenzoyl benzoïque à 96,2% de pureté (83% de rendement chimique). 5 g de cet acide éthyl O.B.B. donnent par chauffage dans l'acide sulfurique 4 g d'éthyl-2 anthraquinone.

Exemple 11

On opère comme à l'exemple 8 mais en remplaçant le benzène par 15,4 g de toluène (0,165 mole). On obtient 30 g d'acide méthyl benzoyl benzoïque (83,4% de rendement chimique). 5 g de cet acide méthyl O.B.B. donnent par chauffage dans l'acide sulfurique 4,1 g de méthyl-2 anthraquinone.

Exemple 12

Dans un autoclave en acier inox on charge à température ordinaire 22,2 g d'anhydride phtalique (0,15 mole) et 10 cm³ de chlorure de méthylène. L'autoclave est refroidi par de l'azote liquide. On introduit 30 g d'HF (1,5 mole) et 102 g de $BF_3$ (1,5 mole). On porte la température à −40°C, introduit en 15 minutes 12,9 g de benzène (0,165 mole), maintient la température une heure à −40°C sous 15 bars, décomprime, dégaze sous vide pour évaporer HF, $BF_3$, et $CH_2Cl_2$ et ajoute de la soude à 10% jusqu'à PH 9. La solution est filtrée puis est acidifiée par addition d'HCl concentré. L'acide O.B.B. précipite sous forme de cristaux blancs très purs. La totalité de l'acide O.B.B. obtenu est traitée pendant 1 heure à 150°C dans de l'acide sulfurique 100%. Après refroidissement et dilution à l'eau on obtient 25,6 g d'anthraquinone à 99,8% soit un rendement chimique global de 82%.

Exemple 13

On opère comme à l'exemple 12 mais en remplaçent le chlorure de méthylène par 15 g de $CO_2$ qui est liquide dans les conditions de la réaction.

On obtient 29,15 g d'acide O.B.B. pur soit un rendement chimique de 86%.

Exemple 14

Dans un autoclave en acier inox on charge à température ordinaire 22,2 g d'anhydride phtalique (0,15 mole) et 17,5 g de paraxylène (0,165 mole).

L'autoclave est refroidi par de l'azote liquide et on introduit 15 g d'HF (0,75 mole) et 102 g de $BF_3$ (1,5 mole).

On laisse la température remonter jusqu'à −40°C et on maintient pendant 30 minutes la température à −40°C sous une pression de 17 bars. On décomprime et on dégaze sous vide pour évaporer HF et $BF_3$ puis on ajoute de la soude 10% jusqu'à PH 9. La solution est filtrée puis est acidifiée par addition d'HCl concentré. L'acide diméthyl-benzoyl-benzoïque précipite sous forme de cristaux blancs très purs.

On obtient 32,9 g de cet acide O.B.B. (86,4% de rendement chimique).

4 g de cet acide diméthyl O.B.B. donnent par chauffage dans de l'acide sulfurique 100% 3,28 g de diméthyl 1,4 anthraquinone.

**Revendications**

1. Procédé pour la préparation des composés anthraquinoniques de formule générale:

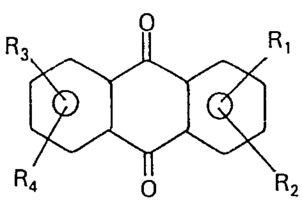

(I)

dans laquelle $R_1$, $R_2$, $R_3$, $R_4$ représente un atome d'hydrogène ou d'halogène ou un groupe alkyle linéaire ou ramifié possédant 1 à 5 atomes de carbone, qui consiste à faire réagir l'anhydride phtalique éventuellement substitué de formule générale:

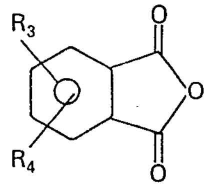

(II)

avec un composé benzénique de formule générale:

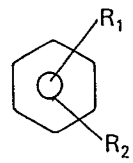

(III)

dans lesquelles $R_1$, $R_2$, $R_3$, $R_4$ ont les mêmes significations que ci-dessus, en présence d'un catalyseur, caractérisé en ce que le catalyseur est constitué par un mélange d'acide fluorhydrique et de trifluorure de bore tel que la quantité de trifluorure de bore est supérieure à 3 moles par mole d'anhydride phtalique, la quantité d'acide fluorhydrique supérieure à 2 moles par mole d'anhydride phtalique, le rapport molaire acide fluorhydrique/trifluorure de bore compris entre 0,2 et 1,5, et en ce que le rapport molaire dérivé aromatique de formule (III)/anhydride phtalique est égal à 0,9 à 1,2, l'acide orthobenzoylbenzoïque ainsi obtenu de formule générale:

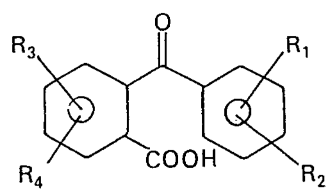

(IV)

dans laquelle $R_1$, $R_2$, $R_3$, $R_4$ ont les mêmes significations que ci-dessus, étant transformé en un composé de formule (I) suivant des procédés connus en soi.

2. Procédé selon la revendication 1, caractérisé en ce que la condensation est effectuée à des températures allant de −60°C à +30°C, la pression allant de 5 à 60 bars.

3. Procédé selon la revendication 2, caractérisé en ce que la condensation a lieu entre 15 et 20 bars et à température de −40°C.

4. Procédé selon la revendication 1, caractérisé en ce que l'acide orthobenzoylbenzoïque de formule (IV) est extrait de la masse réactionnelle par de l'eau à l'ébullition.

5. Procédé selon la revendication 1 caractérisé en ce que l'on utilise 5 à 20 moles de trifluorure de bore et d'acide fluorhydrique par mole d'anhydride phtalique.

**Patentansprüche**

1. Verfahren zur Herstellung von Anthrachinonverbindungen der allgemeinen Formel

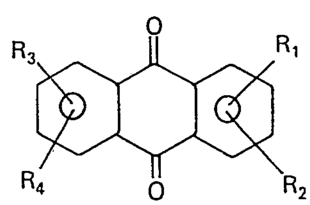

(I)

worin $R_1$, $R_2$, $R_3$, $R_4$ ein Wasserstoffatom oder ein Halogenatom oder eine lineare oder verzweigte Alkylgruppe mit 1 bis 5 Kohlenstoffatomen bedeuten, das darin besteht, dass man gegebenenfalls substituiertes Phthalsäureanhydrid der allgemeinen Formel

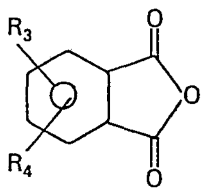

(II)

mit einer Benzolverbindung der allgemeinen Formel

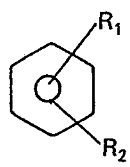

(III)

worin $R_1$, $R_2$, $R_3$, $R_4$ die gleichen Bedeutungen wie oben haben,
in Gegenwart eines Katalysators reagieren lässt, dadurch gekennzeichnet, dass der Katalysator aus einer Mischung von Fluorwasserstoffsäure und Bortrifluorid besteht, so dass die Menge an Bortrifluorid mehr als 3 Mol pro Mol Phthalsäureanhydrid beträgt, die Menge an Fluorwasserstoffsäure mehr als 2 Mol pro Mol Phthalsäureanhydrid beträgt, das Molverhältnis von Fluorwasserstoffsäure zu Bortrifluorid zwischen 0,2 und 1,5 liegt und das Molverhältnis von aromatischem Derivat der Formel (III) zu Phthalsäureanhydrid 0,9 bis 1,2 beträgt,
wonach die erhaltene o-Benzoylbenzoesäure der allgemeinen Formel

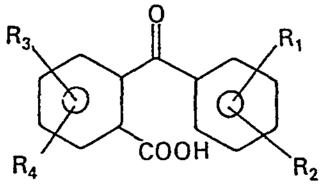

(IV)

worin $R_1$, $R_2$, $R_3$ und $R_4$ die oben angegebenen Bedeutungen haben, nach an sich bekannten Verfahren in eine Verbindung der Formel (I) umgewandelt wird.

2. Verfahren nach Anspruch 1, dadurch gekennzeichnet, dass die Kondensation bei Temperaturen von −60°C bis +30°C und bei einem Druck von 5 bis 60 bar durchgeführt wird.

3. Verfahren nach Anspruch 2, dadurch gekennzeichnet, dass die Kondensation zwischen 15 und 20 bar und bei einer Temperatur von −40°C durchgeführt wird.

4. Verfahren nach Anspruch 1, dadurch gekennzeichnet, dass die o-Benzoylbenzoesäure der Formel (IV) mit siedendem Wasser aus der Reaktionsmasse extrahiert wird.

5. Verfahren nach Anspruch 1, dadurch gekennzeichnet, dass 5 bis 20 Mol Bortrifluorid und Fluorwasserstoffsäure pro Mol Phthalsäureanhydrid verwendet werden.

## Claims

1. Process for the preparation of anthraquinone compounds of general formula:

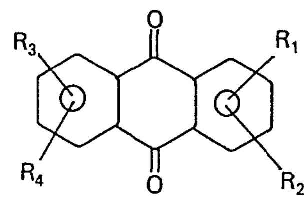

(I)

in which $R_1$, $R_2$, $R_3$, $R_4$ represent an atom of hydrogen or of halogen or a straight or branched alkyl group having 1 to 5 carbon atoms which consists in reacting the phthalic anhydride, possibly substitued, of general formula:

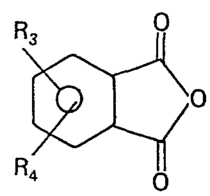

(II)

with a benzene compound of general formula:

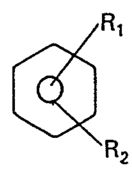

(III)

in which $R_1$, $R_2$, $R_3$, $R_4$ have the same significance as above, in the presence of a catalyst, characterised in that the catalyst consists of a mixture of hydrofluoric acid and boron trifluoride such that the quantity of boron trifluoride is greater than 3 moles per mole of phthalic anhydride, the quantity of hydrofluoric acid is greater than 2 moles per mole of phthalic anhydride, the molar ratio hydrofluoric acid/boron trifluoride is between 0.2 and 1.5, and in that the molar ratio aromatic derivative of formula (III)/phthalic anhydride is equal to 0.9 to 1.2, the orthobenzoyl benzoic acid thus obtained, of general formula:

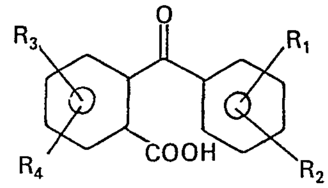

(IV)

in which $R_1$, $R_2$, $R_3$, $R_4$ have the same significance as above, being converted into a compound of formula (I), by processes known in themselves.

2. Process according to claim 1, characterised in that the condensation is effected at temperatures from −60°C to +30°C, the pressure being from 5 to 60 bars.

3. Process according to claim 2, characterised in that the condensation takes place between 15 and 20 bars and at a temperature of −40°C.

4. Process according to claim 1, characterised in that the orthobenzoyl benzoic acid of formula (IV) is extracted from the reaction mass by boiling water.

5. Process according to claim 1, characterised in that 5 to 20 moles of boron trifluoride and of hydrofluoric acid are used per mole of phthalic anhydride.